# EUROPEAN PATENT APPLICATION

(11) **EP 2 647 375 A1**
(43) Date of publication of application: **09.10.2013**
(21) Application number: 13174888.1
(22) Date of filing: 11.05.2010
(51) Int. Cl.: A61K 31/405, A61K 31/4439, A61P 35/00

(54) **Combination of vargatef and dabigatran for the treatment of oncological and fibrotic diseases**

(30) Priority: 14.05.2009 EP 09160203
(62) Divisional of application: 10721441.3
(71) Applicant: BOEHRINGER INGELHEIM INTERNATIONAL GMBH, 55216 Ingelheim am Rhein (DE)
(72) Inventor: Van Ryn, Joanne, 55216 Ingelheim am Rhein (DE); Hilberg, Frank, 55216 Ingelheim am Rhein (DE); Garin-Chesa, Pilar, 55216 Ingelheim am Rhein (DE)
(74) Representative: Simon, Elke Anna Maria

(57) **Abstract**

The invention relates to new methods for the treatment of oncological and fibrotic disease comprising the combined administration of an inhibitor of vascular endothelial growth factor receptors (VEGFRs) in conjunction with a thrombin inhibitor.

## Description

The invention relates to new methods for the treatment of oncological and fibrotic diseases comprising the combined administration of an inhibitor of vascular endothelial growth factor receptors (VEGFRs) in conjunction with a thrombin inhibitor.

### Background to the invention

The compound vargatef (3-Z-[1-(4-(*N*-((4-methyl-piperazin-1-yl)-methylcarbonyl)-*N-*methyl-amino)-anilino)-1-phenyl-methylene]-6-methoxycarbonyl-2-indolinone) is an innovative active ingredient having valuable pharmacological properties, especially for the treatment of oncological and fibrotic diseases, immunologic diseases or pathological conditions involving an immunologic component, or fibrotic diseases. The chemical structure of this compound is depicted below as formula **1**

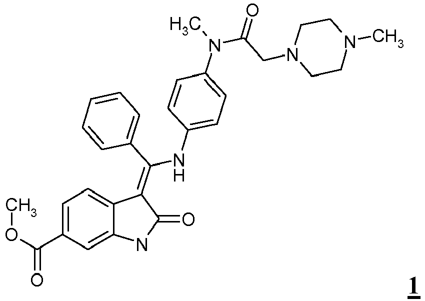

The base form of this compound is described in WO 01/27081, the monoethanesulphonate salt form is described in WO 2004/013099 and various further salt forms are presented in WO 2007/141283. The use of this molecule for the treatment of immunologic diseases or pathological conditions involving an immunologic component is being described in WO 2004/017948 , the use for the treatment of oncological diseases is being described in WO 2004/096224 and the use for the treatment of fibrotic diseases is being described in WO 2006/067165.

The thrombin inhibitor dabigatran of formula **2** 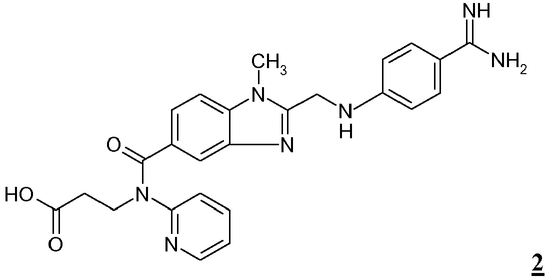 is known from the prior art and was first disclosed in WO98/37075. It is a potent thrombin inhibitor which is known to be usefull for example for the post-operative prevention of deep vein thromboses and in stroke prevention, particularly for preventing strokes in patients with atrial fibrillation.

The purpose of the instant invention is the provision of a new therapy for the treatment of oncological and fibrotic diseases.

### Detailed description of the invention

The invention relates to new methods for the treatment of oncological and fibrotic diseases comprising the combined administration of compound of formula **1** (vargatef) 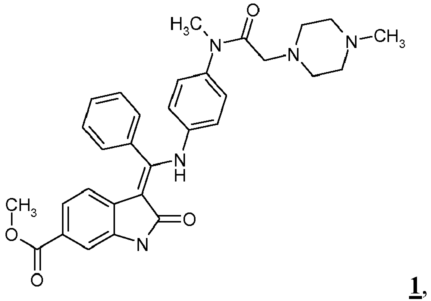 optionally in the form of the tautomers and pharmaceutically acceptable salts thereof, and compound of formula **2** (dabigatran) 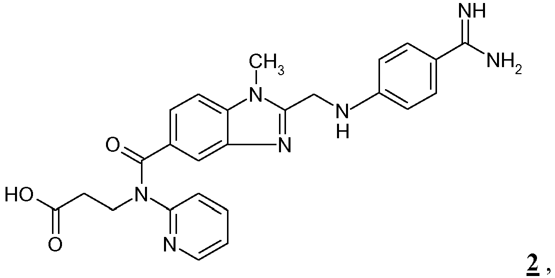 optionally in the form of its prodrugs and the tautomers and pharmaceutically acceptable salts thereof.

According to the instant invention the compounds **1** and **2** can be administered in a single formulation or in two separate formulations. Consequently, in one preferred embodiment the invention relates to pharmaceutical compositions comprising compound **1**, optionally in the form of the tautomers and pharmaceutically acceptable salts thereof, and compound **2**, optionally in the form of its prodrugs and the tautomers and pharmaceutically acceptable salts thereof.

In another preferred embodiment the invention relates to a kit comprising one pharmaceutical composition comprising compound **1**, optionally in the form of the tautomers and pharmaceutically acceptable salts thereof, and a second pharmaceutical composition comprising compound **2,** optionally in the form of its prodrugs and the tautomers and pharmaceutically acceptable salts thereof.

The instant invention is furthermore directed to compound **1**, optionally in the form of the tautomers and pharmaceutically acceptable salts thereof, for use in a method for the treatment of oncological and fibrotic diseases wherein the method furthermore comprises the use of compound **2**, optionally in the form of its prodrugs and the tautomers and pharmaceutically acceptable salts thereof.

The instant invention is furthermore directed to compound **2,** optionally in the form of its prodrugs and the tautomers and pharmaceutically acceptable salts thereof, for use in a method for the treatment of oncological and fibrotic diseases wherein the method furthermore comprises the use of compound **1**, optionally in the form of the tautomers and pharmaceutically acceptable salts thereof.

The instant invention is furthermore directed to the use of compound **1**, optionally in the form of the tautomers and pharmaceutically acceptable salts thereof, for the manufacture of a medicament for the treatment of oncological and fibrotic diseases wherein the treatment furthermore comprises the use of compound **2**, optionally in the form of its prodrugs and the tautomers and pharmaceutically acceptable salts thereof.

The instant invention is furthermore directed to the use of compound **2**, optionally in the form of its prodrugs and the tautomers and pharmaceutically acceptable salts thereof, for the manufacture of a medicament for the treatment of oncological and fibrotic diseases wherein the method furthermore comprises the use of compound **1**, optionally in the form of the tautomers and pharmaceutically acceptable salts thereof.

Within the context of the invention, compound **1** is optionally applied in the form of the tautomers and pharmaceutically acceptable salts thereof. Pharmaceutically acceptable salts are preferably selected from the group consisting of hydrochloride, hydrobromide, hydriodide, hydrosulphate, hydrophosphate, hydromethanesulphonate, hydroethanesulphonate, hydronitrate, hydromaleate, hydroacetate, hydrobenzoate, hydrocitrate, hydrofumarate, hydrotartrate, hydrolactate, hydroxalate, hydrosuccinate, hydrobenzoate and hydro-p-toluenesulphonate, preferably hydrochloride, hydrobromide, hydroethanesulphonate, hydrosulphate, hydrophosphate, hydromaleate, hydrofumarate and hydromethanesulphonate. In a particularly preferred embodiment compound **1** is applied as its hydroethanesulphonate (**1a**) depicted below 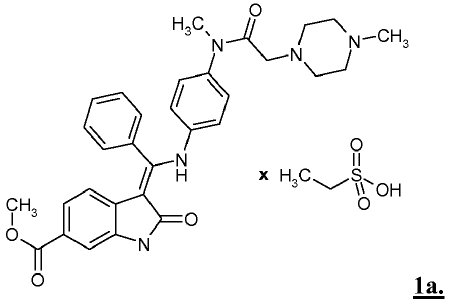 Within the context of this invention the particularly preferred salt of formula **1a** is optionally also referred to as the monoethanesulphonate of compound of formula **1**. The present invention includes the use of the solvates and hydrates of the salts of the compound **1**.

Within the context of the invention, compound **2** is optionally applied in the form of its prodrugs and the tautomers and pharmaceutically acceptable salts thereof. In a particular preferred embodiment compound **2** is applied in the form of its prodrug of formula **2a**, the so-called dabigatran etexilate, 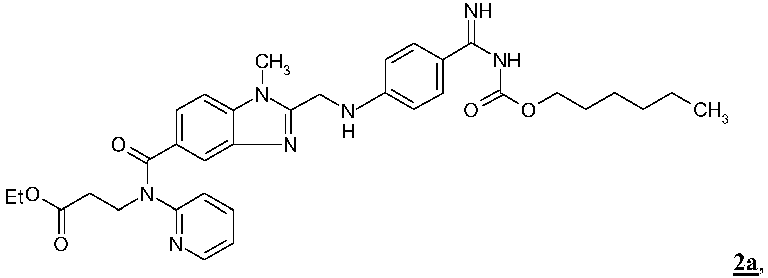 optionally in the form of the tautomers and pharmaceutically acceptable salts thereof.

Pharmaceutically acceptable salts of particularly preferred compound **2a** include acid addition salts which are selected from among the hydrochloride, hydrobromide, hydriodide, hydrosulphate, hydrophosphate, hydromethanesulphonate, hydronitrate, hydromaleate, hydroacetate, hydrobenzoate, hydrocitrate, hydrofumarate, hydrotartrate, hydrolactate, hydroxalate, hydrosuccinate, hydrobenzoate and hydro-p-toluenesulphonate, preferably hydrochloride, hydrobromide, hydrosulphate, hydrophosphate, hydromaleate, hydrofumarate and hydromethanesulphonate. The salts of hydrochloric acid, methanesulphonic acid, maleic acid, benzoic acid and acetic acid are particularly preferred. Of exceptional importance according to the invention are the salts of methanesulphonic acid, which are optionally also referred to as mesylates within the scope of the present invention.

The acid addition salts of compound **2a**, particularly the methanesulphonic acid salt, are disclosed for example in WO 03/074056. The specific polymorphs I and II of the methanesulphonic acid salt or the hemihydrate thereof are also known from the prior art (WO 2005/028468). The present invention includes the use of the solvates and hydrates of the salts of the compound **2a.**

Whereas the main focus of the invention is directed to the combination of compound of formula **1** with compound of formula **2**, the combinations described herein may also be successfully administered in conjunction, with another chomtherapeutic agent, preferably with an inhibitor of the erbB1 receptor (EGFR) and erbB2 (Her2/neu) receptor tyrosine kinases. In a particular preferred embodiment the combination of **1** and **2** is administered together with the compound of formula **3** (tovok) 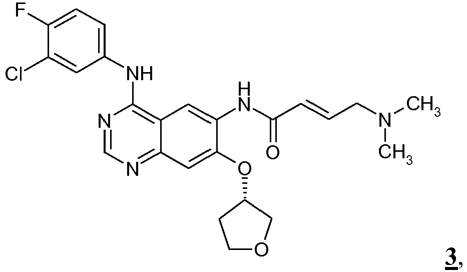 optionally in the form of the tautomers and pharmaceutically acceptable salts thereof. The compound of formula **3** is a potent and selective dual inhibitor of erbB1 receptor (EGFR) and erbB2 (Her2/neu) receptor tyrosine kinases. Furthermore, **3** was designed to covalently bind to EGFR and HER2 thereby irreversibly inactivating the receptor molecule it has bound to. This compound **3,** salts thereof such as the dimaleate salt, their preparation as well as pharmaceutical formulations comprising **3** or a salt thereof, indications to be treated with **3** and combinations including **3** are disclosed in WO 02/50043, WO 2005/037824, WO 2007/054550 and WO 2007/054551.

The combination treatment according to the invention is of particular interest in the treatment of oncological diseases. Preferably the disease is selected from solid tumours, such as urogenital cancers (such as prostate cancer, renal cell cancers, bladder cancers), gynecological cancers (such as ovarian cancers, cervical cancers, endometrial cancers), lung cancer, gastrointestinal cancers (such as non-metastatic or metastatic colorectal cancers, pancreatic cancer, gastric cancer, oesophageal cancers, hepatocellular cancers, cholangiocellular cancers), head and neck cancer (e.g. head and neck squamous cell cancer), malignant glioblastoma, malignant mesothelioma, non-metastatic or metastatic breast cancer (e.g. hormone refractory metastatic breast cancer), malignant melanoma or bone and soft tissue sarcomas, and haematologic neoplasias, such as multiple myeloma, acute myelogenous leukemia, chronic myelogenous leukemia, myelodysplastic syndrome and acute lymphoblastic leukemia. In a preferred embodiment, the disease is non small cell lung cancer (NSCLC), breast cancer (e.g. hormone refractory metastatic breast cancer), head and neck cancer (e.g. head and neck squamous cell cancer), malignant glioblastoma, metastatic colorectal cancers, hormone sensitive or hormone refractory prostate cancer, colorectal cancer, ovarian cancer, hepatocellular cancer, renal cell cancer, soft tissue sarcoma, or small cell lung cancer.

In another embodiment the combination according to the invention is useful for the prevention or treatment of a specific fibrotic disease selected from the group consisting of: Fibrosis and remodeling of lung tissue in chronic obstructive pulmonary disease (COPD), chronic bronchitis, and emphysema; Lung fibrosis and pulmonary diseases with a fibrotic component including but not limited to idiopathic pulmonary fibrosis (IPF), giant cell interstitial pneumonia (GIP), sarcodosis, cystic fibrosis, respiratory distress syndrome (ARDS), granulomatosis, silicosis, drug-induced lung fibrosis (for example, induced by drugs such as bleomycin, bis-chloronitrosourea, cyclophosphamide, amiodarone, procainamide, penicillamine, gold or nitrofurantoin), silicosis, asbestosis, systemic scleroderma; Fibrosis and remodeling in asthma; Fibrosis in rheumatoid arthritis; Virally induced hepatic cirrhosis, for example hepatitis C; Radiation-induced fibrosis; Restenosis, post angioplasty; Renal disorders including chronic glomerulonephritis, renal fibrosis in patients receiving cyclosporine and renal fibrosis due to high blood pressure; Diseases of the skin with a fibrotic component including but not limited to, scleroderma, sarcodosis, systemic lupus erythematosus; Excessive scarring. In a preferred embodiment, the disease is idiopathic pulmonary fibrosis (IPF).

The dosage of the active ingredients in the compositions in accordance with the present invention may be varied, although the amount of the active ingredients **1** and **2** shall be such that a suitable dosage form is obtained. Hence, the selected dosage and the selected dosage form shall depend on the desired therapeutic effect, the route of administration and the duration of the treatment. Suitable dosage ranges for the combination are from the maximal tolerated dose for the single agent to lower doses, e.g. to one tenth of the maximal tolerated dose.
Preferably, between 5 and 1000 mg, particularly preferably 10 to 500 mg of the compound of formula **1** are administered once or several times per day in order to implement the medication according to the invention. Particularly preferably, 25 - 300 mg, more preferably 50 - 200 mg of compound **1** are administered once or twice daily, preferably twice daily.
Preferably, between 50 and 500 mg, particularly preferably 75 to 400 mg of the compound of formula **2a** are administered per day in order to implement the medication according to the invention. Particularly preferably, 110 - 350 mg, more preferably 220 - 300 mg of compound **2a** are administered per day. In a particular preferred dose regimen the method according to the invention comprises administration of 50-200 mg **1** twice daily, preferably 50, 75, 100, 125, 150 or 200 mg **1** twice daily, and 110-150 mg **2a** twice daily.

The foregoing doses are based on the free bases of the compounds **1** and **2**. If the compounds **1** and **2** are applied in the form of their pharmaceutically acceptable salts the amount of the appropriate salt can easily be calculated by the skilled artisan.

For the combination therapy according to the invention the components **1** and **2** may be administered separately (which implies that they are formulated separately) or together (which implies that they are formulated together). Hence, the administration of one element of the combination of the present invention may be prior to, concurrent to, or subsequent to the administration of the other element of the combination. Preferably the components **1** and **2** are administered in different formulations.

As mentioned hereinbefore, the invention relates to pharmaceutical compositions comprising compound **1**, optionally in the form of the tautomers and pharmaceutically acceptable salts thereof, and also compound **2**, optionally in the form of its prodrugs and the tautomers and pharmaceutically acceptable salts thereof. Consequently, if not indicated otherwise throughout the disclosure of this patent application a reference to a combination of **1** and **2** is to be understood as a reference to a combination of compound **1**, optionally in the form of the tautomers and pharmaceutically acceptable salts thereof, and compound **2**, optionally in the form of its prodrugs and the tautomers and pharmaceutically acceptable salts thereof.
The elements of the combination of **1** and **2** may be administered by oral (including buccal or sublingual), enterical, parenteral (e.g., intramuscular, intraperitoneal, intravenous, transdermal or subcutaneous injection, or implant), nasal, vaginal, rectal, or topical (e.g. ocular eyedrops) routes of administration and may be formulated, alone or together, in suitable dosage unit formulations containing conventional non-toxic pharmaceutically acceptable carriers, adjuvants and vehicles appropriate for each route of administration.

In a preferred embodiment the element **1** of the combination in accordance with the invention is administered orally, enterically, transdermally, intravenously, peritoneally or by injection, preferably orally. In another preferred embodiment the component **2** of the combination is preferably administered orally as well. **2** is preferably administered using multiparticulate medicament formulations as described for example in WO 03/074056.

The pharmaceutical compositions for the administration of the components **1** and **2** of this invention may conveniently be presented in dosage unit form and
may be prepared by any of the methods well known in the art of pharmacy. All methods include the step of bringing the active ingredient into association with the carrier which is constituted of one or more accessory ingredients. In general, the
pharmaceutical compositions are prepared by uniformly and intimately bringing the active ingredients into association with a liquid carrier or a finely divided solid carrier or both, and
then, if necessary, shaping the product into the desired dosage form. In the pharmaceutical compositions the active compounds are included in an amount sufficient to produce the desired pharmacologic effect.

The pharmaceutical compositions containing the active ingredients **1** and **2,** separately or together, that are suitable for oral administration may be in the form of discrete units such as hard or soft capsules, tablets, troches or lozenges, each containing a predetermined amount of the active ingredients, or in the form of a dispersible powder or granules, or in the form of a solution or a suspension in an aqueous liquid or non-aqueous liquid, or in the form of syrups or elixirs, or in the form of an oil-in-water emulsion or a water-in-oil emulsion.

Dosage forms intended for oral use may be prepared according to any method known to the art for the manufacture of pharmaceutical formulations and such compositions.

The excipients used may be, for example: (a) inert diluents such as mannitol, sorbitol, calcium carbonate, pregelatinized starch, lactose, calcium phosphate or sodium phosphate; (b) granulating and disintegrating agents, such as povidone, copovidone, hydroxypropylmethylcellulose, corn starch, alginic acid, crospovidone, sodiumstarchglycolate, croscarmellose, or polacrilin potassium ; (c) binding agents such as microcrystalline cellulose or acacia ; and (d) lubricating agents such as magnesium stearate, stearic acid, fumaric acid or talc.

In some cases, formulations for oral use may be in the form of hard gelatin or HPMC (hydroxypropylmethylcellulose) capsules wherein the active ingredients **1** or **2**, separately or together, is mixed with an inert solid diluent, for example pregelatinized starch, calcium carbonate, calcium phosphate or kaolin, or dispensed via a pellet formulation. They may also be in the form of soft gelatin capsules wherein the active ingredient is mixed with water or an oil medium, for example peanut oil, liquid paraffin, medium chain triglycerides or olive oil.

The tablets, capsules or pellets may be uncoated or they may be coated by known techniques to delay disintegration and absorption in the gastrointestinal tract and thereby provide a delayed action or sustained action over a longer period. For example, a time delay material such as celluloseacetate phtalate or hydroxypropylcellulose acetate succinate or sustained release material such as ethylcellulose or ammoniomethacrylate copolymer (type B) may be employed.

Liquid dosage forms for oral administration in accordance with the present invention include pharmaceutically acceptable emulsions, solutions, suspensions, syrups, and elixirs containing inert diluents commonly used in the art, such as water. Besides such inert diluents, compositions can also include adjuvants, such as wetting agents, emulsifying and suspending agents, and sweetening, flavoring, perfuming and preserving agents.

Aqueous suspensions in accordance with the present invention normally contain the active materials **1** and **2**, separately or together, in admixture with excipients suitable for the manufacture of aqueous suspensions. Such excipients may be (a) suspending agents such as hydroxy ethylcellulose, sodium carboxymethylcellulose, methylcellulose, hydroxypropylmethylcellulose, sodium alginate, polyvinylpyrrolidone, gum tragacanth and gum acacia; (b) dispersing or wetting agents which may be (b.1) a naturally-occurring phosphatide such as lecithin, (b.2) a condensation product of an alkylene oxide with a fatty acid, for example, polyoxyethylene stearate, (b.3) a condensation product of ethylene oxide with a long chain aliphatic alcohol, for example heptadecaethyleneoxycetanol, (b.4) a condensation product of ethylene oxide with a partial ester derived from a fatty acid and a hexitol such as polyoxyethylene sorbitol monooleate, or (b.5) a condensation product of ethylene oxide with a partial ester derived from a fatty acid and a hexitol anhydride, for example polyoxyethylene sorbitan monooleate.

The aqueous suspensions may also contain: one or more preservatives, for example, ethyl or n-propyl p-hydroxybenzoate; one or more coloring agents; one or more flavoring agents; and one or more sweetening agents, such as sucrose or saccharin.

Oily suspensions in accordance with the present invention may be formulated by suspending the active ingredients **1** and **2**, separately or together, in a vegetable oil, for example arachis (peanut) oil, olive oil, sesame oil or coconut oil, or in a mineral oil such as liquid paraffin. The oily suspensions may contain a thickening agent, for example beeswax, hard paraffin or cetyl alcohol. Sweetening agents and flavoring agents may be added to provide a palatable oral preparation. These compositions may be prepared by the addition of an antioxidant such as ascorbic acid.

Dispersible powders and granules are suitable formulations for the preparation of an aqueous suspension in accordance with the present invention. In these formulations the active ingredients **1** and **2** are present, separately or together, in admixture with a dispersing or wetting agent, a suspending agent and one or more preservatives. Suitable examples of dispersing or wetting agents, suspending agents and preservatives are those already mentioned hereinbefore. Additional excipients such as, for example, sweetening, flavouring and colouring agents may also be present. Suitable examples of excipients are those already mentioned hereinbefore.

The pharmaceutical compositions of the invention may also be in the form of oil-in-water emulsions. The oily phase may be a vegetable oil such as olive oil or arachis (peanut) oil, or a mineral oil such as liquid paraffin or a mixture thereof.

Suitable emulsifying agents may be (a) naturally-occurring gums such as gum acacia and gum tragacanth, (b) naturally-occurring phosphatides such as soybean and lecithin, (c) esters or partial esters derived from fatty acids and hexitol anhydrides, for example sorbitan monooleate, (d) condensation products of said partial esters with ethylene oxide, for example polyoxyethylene sorbitan monooleate. The emulsions may also contain sweetening and flavouring agents.

Syrups and elixirs in accordance with the present invention may be formulated with sweetening agents, for example glycerol, propylene glycol, sorbitol or sucrose. Such formulations may also contain a preservative and flavoring and coloring agents.

The pharmaceutical compositions containing **1** and **2**, separately or together, may be in the form of a sterile injectable aqueous or oleagenous suspension or solution. The suspension may be formulated according to known methods using those suitable dispersing or wetting agents and suspending agents which have been mentioned hereinbefore. A suitable sterile injectable preparation may also be a sterile injectable solution or suspension in a non toxic parenterally-acceptable diluent or solvent, for example a solution in 1,3-butane-diol. Examples of suitable acceptable vehicles and solvents that may be employed are water, Ringer's solution and an isotonic sodium chloride solution. In addition, sterile, fixed oils may conventionally be employed as a solvent or suspending medium. For this purpose any bland fixed oil may be employed, including synthetic mono-or diglycerides. In addition, fatty acids such as oleic acid find use in the preparation of injectables in accordance with the present invention.

Preparations for parenteral administration according to the present invention containing **1** and **2,** separately or together, include sterile aqueous or non-aqueous solutions, suspension, or emulsions.

Examples of suitables non-aqueous solvents or vehicles for the preparations in accordance with the present invention are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, by filtration through a bacteria-retaining filter, by incorporating sterilizing agents into the compositions, by irradiating the compositions, or by heating the compositions. They may also be manufactured in the form of sterile solid compositions which can be reconstituted in sterile water, or some other sterile injectable medium immediately before use.

The elements **1** and **2** of the combination of this invention may also be administered in the form of suppositories for rectal administration. Such compositions can be prepared by mixing the active ingredient with a suitable non-irritating excipient which is solid at ordinary temperatures but liquid at the rectal temperature and will therefore melt in the rectum to release the active ingredient. Such materials are cocoa butter, hard fat, and polyethylene glycols.

Compositions for buccal, nasal or sublingual administration in accordance with the present invention may be prepared with standard excipients well known in the art.

For topical administration, the elements **1** and **2** of the combination of this invention may be formulated, separately or together, in liquid or semi-liquid preparations. Examples of suitable preparations are: liniments, lotions, applications; oil-in-water or water-in-oil emulsions such as creams, ointments, jellies or pastes, including tooth-pastes; solutions or suspensions such as drops.

In a preferred embodiment, the active ingredient **1** or a pharmaceutically acceptable salt thereof is formulated in the form of a capsule such as for example a hard gelatin or a hydroxypropylmethylcellulose (HPMC) capsule, comprising a capsule shell and a capsule formulation, wherein the capsule formulation comprises a suspension of the active ingredient **1** or a pharmaceutically acceptable salt thereof, preferably a viscous suspension comprising a carrier and a thickener, more preferably a viscous suspension in which the carrier is a lipid (lipophilic) carrier.

The compound 2 is preferably administered using multiparticulate medicament formulations as described for example in WO 03/074056. Figure 1 of WO 03/74056 shows the schematic structure of preferred pharmaceutical compositions by means of a section through a suitable pellet. The approximately ball-shaped/spherical core region of this pellet contains or consists of a pharmaceutically acceptable organic acid, preferably tartaric acid. Then comes a layer that separates the acid core from the layer containing the active substance, the so-called isolating layer. The isolating layer is in turn surrounded by the active substance layer, which is also in the shape of a spherical shell, which in turn may be surrounded by a coating that improves the abrasion resistance and storage stability of the pellets.

The preparation of pellet formulations of this kind that are preferably used according to the invention is characterised by a series of partial steps. First, the core is prepared from pharmaceutically acceptable organic acid. Within the scope of the present invention tartaric acid is used to prepare the core. The core material thus obtained is then converted into so-called isolated tartaric acid cores by spraying on an isolating suspension. A dabigatran suspension prepared subsequently is sprayed onto these coated cores by means of a coating process in one or more process steps. The active substance pellets thus obtained are then packed into suitable capsules.

The experimental section that follows summarises the preparation of the medicament formulations that are particularly preferably used according to the invention.

### Experimental Part

### A) Preferred Examples of Dosage Forms comprising 1:

The tables below show pharmaceutical compositions for **1**.

The active substance in all the Examples is 3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethanesulphonate.

### Example 1

**Soft gelatin capsule containing 50 mg of active substance**

| | | **Formulation A** | **Formulation B** | **Formulation C** |
|---|---|---|---|---|
| **Ingredients** | **Function** | **mg per capsule** | **mg per capsule** | **mg per capsule** |
| Active Substance* | Active Ingredient | 60.20 | 60.20 | 60.20 |
| Triglycerides, Medium-chain | Carrier | 40.95 | 53.70 | 54.00 |
| Hard fat | Thickener | 38.25 | 25.50 | 25.50 |
| Lecithin | Wetting agent / Glidant | 0.60 | 0.60 | 0.30 |
| Gelatin | Film-former | 72.25 | 72.25 | 72.25 |
| Glycerol 85% | Plasticizer | 32.24 | 32.24 | 32.24 |
| Titanium dioxide | Colorant | 0.20 | 0.20 | 0.20 |
| Iron oxide A | Colorant | 0.32 | 0.32 | 0.32 |
| Iron oxide B | Colorant | 0.32 | 0.32 | 0.32 |
| **Total Capsule Weight** | | **245.33** | **245.33** | 245.33 |

| | | | | |
|---|---|---|---|---|
| * The figures refer to the amount of ethanesulfonate salt (dry basis) equivalent to the labeled amount of the free base | | | | |

### Example 1a

**Soft gelatin capsule containing 75 mg of active substance**

| | | **Formulation A** | **Formulation B** | **Formulation C** |
|---|---|---|---|---|
| **Ingredients** | **Function** | **mg per capsule** | **mg per capsule** | **mg per capsule** |
| Active Substance* | Active Ingredient | 90.3 | 90.3 | 90.3 |
| Triglycerides, Medium-chain | Carrier | 61.425 | 80.55 | 80.1 |
| Hard fat | Thickener | 57.375 | 38.25 | 38.25 |
| Lecithin | Wetting agent / Glidant | 0.9 | 0.9 | 1.35 |
| Gelatin | Film-former | 107.11 | 107.11 | 107.11 |
| Glycerol 85% | Plasticizer | 46.84 | 46.84 | 46.84 |
| Titanium dioxide | Colorant | 0.35 | 0.35 | 0.35 |
| Iron oxide A | Colorant | 0.058 | 0.058 | 0.058 |
| Iron oxide B | Colorant | 0.16 | 0.16 | 0.16 |
| **Total Capsule Weight** | | **364.518** | **364.518** | **364.518** |

| | | | | |
|---|---|---|---|---|
| * The figures refer to the amount of ethanesulfonate salt (dry basis) equivalent to the labeled amount of the free base | | | | |

### Example 2

**Soft gelatin capsule containing 100 mg of active substance**

| | | **Formulation A** | **Formulation B** | **Formulation C** |
|---|---|---|---|---|
| **Ingredients** | **Function** | **mg per capsule** | **mg per capsule** | **mg per capsule** |
| Active Substance* | Active Ingredient | 120.40 | 120.40 | 120.40 |
| Triglycerides, Medium-chain | Carrier | 81.90 | 107.40 | 106.8 |
| Hard fat | Thickener | 76.50 | 51.00 | 51.00 |
| Lecithin | Wetting agent / Glidant | 1.20 | 1.20 | 1.80 |
| Gelatin | Film-former | 111.58 | 111.58 | 111.58 |
| Glycerol 85% | Plasticizer | 48.79 | 48.79 | 48.79 |
| Titanium dioxide | Colorant | 0.36 | 0.36 | 0.36 |
| Iron oxide A | Colorant | 0.06 | 0.06 | 0.06 |
| Iron oxide B | Colorant | 0.17 | 0.17 | 0.17 |
| **Total Capsule Weight** | | **440.96** | **440.96** | **440.96** |

| | | | | |
|---|---|---|---|---|
| * The figures refer to the amount of ethanesulfonate salt (dry basis) equivalent to the labeled amount of the free base | | | | |

### Example 3

**Soft gelatin capsule containing 125 mg of active substance**

| | | **Formulation A** | **Formulation B** | **Formulation C** |
|---|---|---|---|---|
| **Ingredients** | **Function** | **mg per capsule** | **mg per capsule** | **mg per capsule** |
| Active Substance* | Active Ingredient | 150.50 | 150.50 | 150.50 |
| Triglycerides, Medium-chain | Carrier | 102.375 | 134.25 | 133.5 |
| Hard fat | Thickener | 95.625 | 63.75 | 63.75 |
| Lecithin | Wetting agent / Glidant | 1.50 | 1.50 | 2.25 |
| Gelatin | Film-former | 142.82 | 142.82 | 142.82 |
| Glycerol 85% | Plasticizer | 62.45 | 62.45 | 62.45 |
| Titanium dioxide | Colorant | 0.47 | 0.47 | 0.47 |
| Iron oxide A | Colorant | 0.08 | 0.08 | 0.08 |
| Iron oxide B | Colorant | 0.22 | 0.22 | 0.22 |
| **Total Capsule Weight** | | **556.04** | **556.04** | **556.04** |

| | | | | |
|---|---|---|---|---|
| * The figures refer to the amount of ethanesulfonate salt (dry basis) equivalent to the labeled amount of the free base | | | | |

### Example 4

**Soft gelatin capsule containing 150 mg of active substance**

| | | **Formulation A** | **Formulation B** | **Formulation C** |
|---|---|---|---|---|
| **Ingredients** | **Function** | **mg per capsule** | **mg per capsule** | **mg per capsule** |
| Active Substance* | Active Ingredient | 180.60 | 180.60 | 180.60 |
| Triglycerides, Medium-chain | Carrier | 122.85 | 161.10 | 160.20 |
| Hard fat | Thickener | 114.75 | 76.50 | 76.50 |
| Lecithin | Wetting agent / Glidant | 1.80 | 1.80 | 2.70 |
| Gelatin | Film-former | 142.82 | 142.82 | 142.82 |
| Glycerol 85% | Plasticizer | 62.45 | 62.45 | 62.45 |
| Titanium dioxide | Colorant | 0.47 | 0.47 | 0.47 |
| Iron oxide A | Colorant | 0.08 | 0.08 | 0.08 |
| Iron oxide B | Colorant | 0.22 | 0.22 | 0.22 |
| **Total Capsule Weight** | | **626.04** | **626.04** | **626.04** |

| | | | | |
|---|---|---|---|---|
| * The figures refer to the amount of ethanesulfonate salt (dry basis) equivalent to the labeled amount of the free base | | | | |

### Example 5

**Soft gelatin capsule containing 200 mg of active substance**

| | | **Formulation A** | **Formulation B** | **Formulation C** |
|---|---|---|---|---|
| **Ingredients** | **Function** | **mg per capsule** | **mg per capsule** | **mg per capsule** |
| Active Substance* | Active Ingredient | 240.80 | 240.80 | 240.80 |
| Triglycerides, Medium-chain | Carrier | 163.30 | 214.80 | 216.00 |
| Hard fat | Thickener | 153.50 | 102.00 | 102.00 |
| Lecithin | Wetting agent / Glidant | 2.40 | 2.40 | 1.20 |
| Gelatin | Film-former | 203.19 | 203.19 | 203.19 |
| Glycerol 85% | Plasticizer | 102.61 | 102.61 | 102.61 |
| Titanium dioxide | Colorant | 0.57 | 0.57 | 0.57 |
| Iron oxide A | Colorant | 0.90 | 0.90 | 0.90 |
| Iron oxide B | Colorant | 0.90 | 0.90 | 0.90 |
| **Total Capsule Weight** | | **868.17** | **868.17** | **868.17** |

| | | | | |
|---|---|---|---|---|
| * The figures refer to the amount of ethanesulfonate salt (dry basis) equivalent to the labeled amount of the free base | | | | |

### Example 6

The table below shows further pharmaceutical compositions according to the invention. D, E and F are tablets, G can be compressed to form tablets after hot melt-granulation of the active substance in a heated/ cooled high-shear mixer together with Microcrystalline cellulose an Macrogol 6000. After further mixing steps of the obtained granules with the other excipients, tablets are produced on a conventional tablet press. Alternatively it can be directly dispensed as oral granules into sachets.

Tablet D and F may be produced by direct blending of the components and subsequent compression on a conventional tablet press. Alternatively it can be extruded to pellets and filled into a hard capsule.
Tablet E may be produced by wet granulation of the drug substance together with Lactose monohydrate and Microcrystalline cellulose by an aqueous solution of Copovidone. After further blending steps with Crospovidone, Colloidal silica and Magnesium stearate, the tablets are compressed on a conventional tablet press.

| **Formulation** | **D** | **E** | **F** | **G** | **H** | **I** |
|---|---|---|---|---|---|---|
| Active Substance* | 180.6 mg | 150.5 mg | 120.4 mg | 150.5 mg | 60.2 mg | 60.2 mg |
| Sorbitol | - | - | - | - | - | 125.0 mg |
| Lactose monohydrate | 50.0 mg | 125.0 mg | - | - | - | - |
| Microcrystalline cellulose | - | 20.0 mg | 150.0 mg | 80.0 mg | - | 20.0 mg |
| Calcium phopsphate | 30.0 mg | - | 150.0 mg | - | - | - |
| Soybean Oil | - | - | - | - | 145.0 mg | - |
| Macrogol 6000 | - | - | - | 80.0 mg | - | - |
| Copovidone | 2.0 mg | 10.0 mg | - | - | - | - |
| Sodium starch glycolate | 5.0 mg | - | - | - | - | - |
| Crospovidone | - | 5.0 mg | 5.0 mg | - | - | 5.0 mg |
| Cremophor RH 40 | - | - | - | - | 20.0 mg | - |
| Colloidal silica | 1.0 mg | 1.0 mg | 1.0 mg | - | 10.0 mg | 1.0 mg |
| Solid flavour | - | - | - | 5.0 mg | - | 4.0 mg |
| Magnesium stearate | 4.0 mg | 4.0 mg | 4.0 mg | - | - | - |
| Total | 272.6 mg | 315.5 mg | 430.4 mg | 315.5 mg | 235.2 mg | 215.2 mg |

| | | | | | | |
|---|---|---|---|---|---|---|
| * The figures refer to the amount of ethanesulfonate salt **1a** | | | | | | |

Formulation H is prepared as a liquid fillmix of suspended active. After homogenization it is filled either in hard or soft gelatine capsules. Formulation I is an oral powder.

### B) Preferred Examples of Dosage Forms comprising 2:

In the following section the manufacturing method for preferred dosage forms of **2** is discribed.

### B.1 - Preparation of the Starter Pellets

480kg water are heated to 50°C and 120 kg of acacia (gum arabic) are added with stirring in a conventional mixing container having a dished end and stirrer. Stirring is continued at constant temperature until a clear solution is obtained. Once there is a clear solution (usually after 1 to 2 hours) 600 kg tartaric acid are added with stirring. The tartaric acid is added at constant temperature and while stirring is continued. After the addition has ended the mixture is stirred for about another 5 to 6 hours.

1000 kg tartaric acid are added to a slowly rotating (3 revolutions per minute) unperforated horizontal pan with a spraying and powder applying unit (e.g. Driamat 2000/2.5). Before spraying starts, a sample of the acid is taken for screening analysis. The acid in question is tartaric acid particles with a particle size in the range from 0.4-0.6 mm.
The acid rubber solution obtained by the above method is sprayed onto the tartaric acid particles thus provided. During the spraying, the quantity of air supplied is adjusted to 1000m³/h and 35°-75°C. The differential pressure is 2 mbar and the speed of rotation of the pan is 9 revolutions per minute. The nozzles should be arranged at a distance of 350 - 450mm from the filling.

The acid rubber solution is sprayed on by alternating with the following steps. After about 4.8 kg of the acid rubber solution has been sprayed onto the tartaric acid particles of particle size 0.4-0.6 mm and the solution has been distributed, about 3.2 kg tartaric acid powder are sprinkled onto the damp tartaric acid particles. The tartaric acid powder in question consists of fine tartaric acid particles with a particle size of < 50 microns. In all, 800 kg tartaric acid powder are required. After the said tartaric acid powder has been sprinkled on and distributed the spray material is dried until a product temperature of about 40°C is reached. This is in turn followed by the spraying on of the acid rubber solution.

These cycles are repeated until the acid rubber solution is used up. Once the process has ended the acid pellets are dried in the pan at 3 rpm for 240 minutes. To prevent caking after the drying has finished, an intermittent program is run at 3 rpm for 3 minutes every hour. In the present instance this means that the pan is rotated at 3 rpm for 3 minutes at intervals of one hour and then left to stand. The acid pellets are then transferred into a drying apparatus. They are then dried at 60°C over a period of 48 hours. Finally, the particle size distribution is determined by screen analysis. The particle size with a diameter of 0.6 - 0.8mm corresponds to the product. This fraction should make up >85%.

### B.2 - Isolation of the Starter Pellets

To prepare the isolating suspension, 666.1 (347.5) kg of ethanol are placed in the mixing container and the hydroxypropylmethylcellulose (33.1 (17.3) kg) is added with stirring at approx. 600 rpm and dissolved. Then under the same conditions 0.6 (0.3) kg dimeticone are added. Shortly before use, talc (33.1 (17.3) kg) is added, again with stirring, and suspended.

The acid pellets 1200 (600) kg are poured into the coating apparatus (e.g. GS- Coater Mod. 600/Mod. 1200) and sprayed therein in the rotating pan with the isolating suspension described above in a continuous spraying process lasting several hours at a spraying rate of 32 kg/h for the 1200 kg mixture or 21 kg/h for the 600 kg mixture. The pellets are also dried continuously with an air supply at up to 70°C.

After the GS Coater has been emptied, the isolated starter pellets are fractionated by screening. The product fraction with a diameter ≤1.0 mm is stored and used further.

### B.3 - Preparation of the dabigatran etexilate 2a suspension

26.5 kg hydroxypropylcellulose are added to 720 kg isopropanol in a 1200 litre mixing container fitted with a propeller stirrer and the mixture is stirred until fully dissolved (about 12 - 60 hours; roughly 500 rpm). Once the solution is clear, 132.3 kg of dabigatran etexilate methanesulphonate (polymorph I) are added with stirring (400 rpm) and the mixture is stirred for about another 20-30 minutes. Then 21.15 kg of talc is added at a constant stirring rate and stirring is continued at the same speed for about another 10-15 minutes. The steps described above are preferably carried out under a nitrogen atmosphere.

Any clumps formed are broken up by homogenising using an UltraTurrax stirrer (about 60-200 minutes). The suspension temperature should not exceed 30°C throughout the entire manufacturing process.

The suspension is stirred until ready for further processing to ensure that no sedimentation occurs (at roughly 400 rpm).

If the suspension is stored at below 30°C, it should be further processed within at most 48 h. If for example the suspension is manufactured and stored at 22°C, it should be further processed within 60 hours.

### B.4 - Preparation of the dabigatran etexilate active substance pellets

A horizontal pan with an unperforated container is used (GS Coater Mod. 600). In contrast to the fluidised bed method, the suspension is sprayed onto the fluidised bed of pellets in the rotating pan by the "top spray" method. It is sprayed on through nozzles 1.4 mm in diameter. The dry air is passed into the bed of pellets through so-called immersion blades and transported away through an opening in the back wall of the coater.

The horizontal pan is charged with 320 kg of the tartaric acid pellets obtained according to B.2 and the bed of pellets is heated up. Once a product temperature of 43°C has been reached, spraying begins. 900 kg of the suspension prepared previously according to B.3 are sprayed on, first of all for 2 h at a spraying rate of 20 kg/h, then 24 kg/h. The suspension is stirred constantly. The temperature of the air supplied is at most 75°C. The amount of air supplied is about 1900m³/h.

Then the pellets are dried in the horizontal pan (5 revolutions per minute) at an air inflow temperature of at least 30°C, at most 50°C and an air inflow amount of 500 m³/h over a period of about 1-2 hours.

325 kg of the pellets thus obtained are then loaded once more into a horizontal pan and heated to 43°C. 900 kg of the suspension prepared previously according to B.3 are sprayed on, first of all for 2 h at a spraying rate of 20 kg/h, then 24 kg/h. The suspension is stirred constantly. The temperature of the air supplied is at most 75°C. The amount of air supplied is about 1900 m³/h.

Then the pellets are dried in the horizontal pan (5 revolutions per minute) at an air inflow temperature of at least 30°C, at most 50°C and an air inflow amount of 500 m³/h over a period of about 1-2 hours.

The dried pellets are then passed through a vibrating screen with a mesh size of 1.6 mm and stored in containers with desiccants until needed for further processing.

### B.5 - Examples of formulations comprising 2

The following examples of formulations are then obtained from the active substance pellets obtained according to B.4 by packing into hydroxypropylmethylcellulose capsules:

| **Ingredient** | **amount [mg] per capsule** | **amount [mg] per capsule** |
|---|---|---|
| active substance **I** | 86.48⁽¹⁾ | 126.83⁽²⁾ |
| Acacia (gum arabic) | 4.43 | 6.50 |
| tartaric acid | 88.56 | 129.9 |
| hydroxymethyl-propylcellulose 2910 | 2.23 | 3.27 |
| dimethylpolysiloxane 350 | 0.04 | 0.06 |
| talc | 17.16 | 25.16 |
| hydroxypropylcellulo se | 17.30 | 25.37 |
| HPMC capsule | 60⁽³⁾ | 70⁽⁴⁾ |
| Total | 276.2 | 387.1 |

| | | |
|---|---|---|
| ⁽¹⁾ corresponds to 75 mg of free active substance base **2a** ⁽²⁾ corresponds to 110 mg of free active substance base **2a** ⁽³⁾ weight of capsule size is about 60 mg ⁽⁴⁾ weight of capsule size is about 70 mg | | |

## Claims

1. Pharmaceutical composition comprising a compound of formula **1** 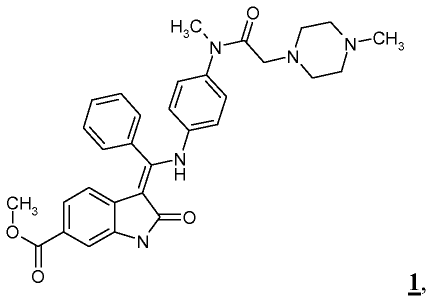 optionally in the form of the tautomers and pharmaceutically acceptable salts thereof, and a compound of formula **2** 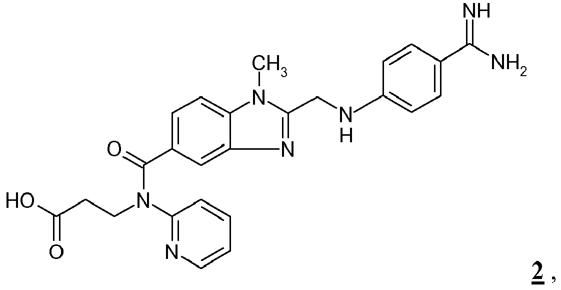 optionally in the form of its prodrugs and the tautomers and pharmaceutically acceptable salts thereof.

2. Pharmaceutical composition according to claim 1, wherein compounds **1**, optionally in the form of the tautomers and pharmaceutically acceptable salts thereof , and 2, optionally in the form of its prodrugs and the tautomers and pharmaceutically acceptable salts thereof, are administered in two separate formulations.

3. Pharmaceutical compositions according to one of claims 1 or 2, wherein **1**, optionally in the form of its tautomers, is present in the form of one of its pharmaceutically acceptable salts that are preferably selected from the group consisting of hydrochloride, hydrobromide, hydriodide, hydrosulphate, hydrophosphate, hydromethanesulphonate, hydroethanesulphonate, hydronitrate, hydromaleate, hydroacetate, hydrobenzoate, hydrocitrate, hydrofumarate, hydrotartrate, hydrolactate, hydroxalate, hydrosuccinate, hydrobenzoate and hydro-p-toluenesulphonate, preferably hydrochloride, hydrobromide, hydroethanesulphonate, hydrosulphate, hydrophosphate, hydromaleate, hydro fumarate and hydromethanesulphonate.

4. Pharmaceutical compositions according to one of claims 1 to 3, wherein compound **1**, optionally in the form of its tautomers, is applied as its hydroethanesulphonate (**1a**)

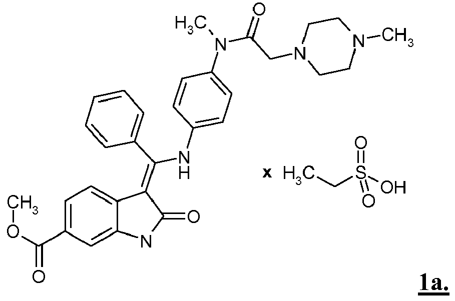

5. Pharmaceutical compositions according to one of claims 1 to 4, wherein compound **2** is applied in form of its prodrug of formula **2a**, 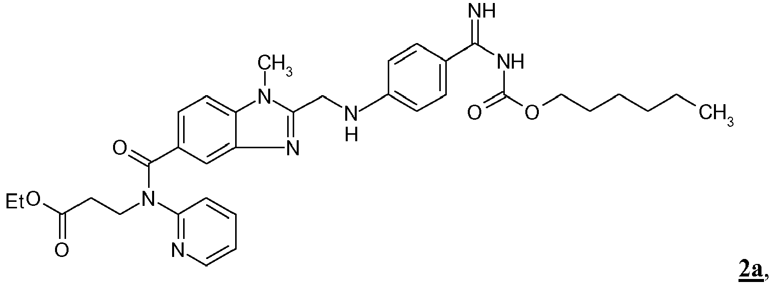 optionally in the form of the tautomers and pharmaceutically acceptable salts thereof.

6. Pharmaceutical compositions according to claim 5, wherein **2a** is applied in the form of one of its pharmaceutically acceptable salts that are preferably selected from among the hydrochloride, hydrobromide, hydriodide, hydrosulphate, hydrophosphate, hydromethanesulphonate, hydronitrate, hydromaleate, hydroacetate, hydrobenzoate, hydrocitrate, hydrofumarate, hydrotartrate, hydrolactate, hydroxalate, hydrosuccinate, hydrobenzoate and hydro-p-toluenesulphonate, preferably hydrochloride, hydrobromide, hydrosulphate, hydrophosphate, hydromaleate, hydrofumarate and hydromethanesulphonate, preferably hydrochloride, hydromethanesulphonate, hydromaleate, hydrobenzoate and hydroacetate.

7. Pharmaceutical compositions according to claim 5 or 6, wherein **2a** is applied in the form of its hydromethanesulphonate.

8. Pharmaceutical compositions according to one of claims 1 to 7, further comprising a compound of formula **3** 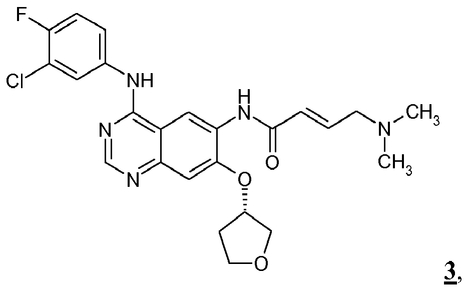 optionally in the form of the tautomers and pharmaceutically acceptable salts thereof.

9. Kit comprising one pharmaceutical composition comprising compound **1**, optionally in the form of the tautomers and pharmaceutically acceptable salts thereof, and another pharmaceutical composition comprising compound **2**, optionally in the form of its prodrugs and the tautomers and pharmaceutically acceptable salts thereof.

10. Pharmaceutcal compositions according to one of claims 1 to 9 for the treatment of oncological and fibrotic diseases.

11. Pharmaceutical compositions according to one of claims 1 to 9 for the treatment of a disease selected from solid tumours, urogenital cancers, gynecological cancers, lung cancer, gastrointestinal cancers, head and neck cancer, malignant glioblastoma, malignant mesothelioma, non-metastatic or metastatic breast cancer, malignant melanoma or bone and soft tissue sarcomas, and haematologic neoplasias, such as multiple myeloma, acute myelogenous leukemia, chronic myelogenous leukemia, myelodysplastic syndrome and acute lymphoblastic leukemia.

12. Use of a pharmaceutical composition according to one of claims 1 to 9 for the manufacture of a medicament for the treatment of oncological and fibrotic diseases.

13. Use according to claim 12, wherein the diseases is selected from solid tumours, urogenital cancers, gynecological cancers, lung cancer, gastrointestinal cancers, head and neck cancer, malignant glioblastoma, malignant mesothelioma, non-metastatic or metastatic breast cancer, malignant melanoma or bone and soft tissue sarcomas, and haematologic neoplasias, such as multiple myeloma, acute myelogenous leukemia, chronic myelogenous leukemia, myelodysplastic syndrome and acute lymphoblastic leukemia.
